# EUROPEAN PATENT SPECIFICATION

(11) **EP 3 592 323 B1**
(45) Date of publication and mention of the grant of the patent: **17.02.2021**
(21) Application number: 17712041.7
(22) Date of filing: 08.03.2017
(51) Int. Cl.: A61C 13/00, A61C 5/20, A61C 5/77, A61K 6/833, C03C 3/097, C03C 4/00, C03C 10/00

(54) **METHOD TO PRODUCE A MONOLITHIC FORM BODY**
VERFAHREN ZUR HERSTELLUNG EINES MONOLITHISCHEN FORMKÖRPERS
PROCÉDÉ DE PRODUCTION D'UN CORPS À FORME MONOLITHIQUE

(43) Date of publication of application: 15.01.2020
(73) Proprietor: Dentsply Sirona Inc., York, PA 17401 (US); DeguDent GmbH, 63457 Hanau (DE)
(72) Inventor: VOLLMANN, Markus, 63571 Gelnhausen (DE)
(74) Representative: Dietz, Mirko
(86) International application number: PCT/EP2017/055438
(87) International publication number: WO 2018/162055

(56) References cited:
- WO-A1-2016/188914
- GB-A- 1 589 617
- US-A1- 2006 136 071
- US-A1- 2013 026 157

## Description

The invention relates to a method for producing a monolithic form body, in particular a dental restoration, such as a bridge, crown, coping, inlay, onlay or veneer, of lithium silicate glass ceramic.

The use of lithium silicate glass ceramic has proven itself in the field of dental technology because of its strength and biocompatibility. The strength can additionally be increased by adding a stabilizer from the group consisting of zirconium oxide, hafnium oxide, or mixtures thereof, to the starting raw materials (DE 10 2009 060 274 A1, WO 2012/175450 A1, WO 2012/175615 A1, WO 2013/053865 A2, EP 2 662 342 A1).

Lithium silicate glass ceramic materials, in particular if a blank contains lithium metasilicate as the main crystal phase, enable problem-free machining without high tool wear. To increase the strength, the lithium metasilicate is at least partially converted into lithium disilicate by subsequent heat treatment (DE 197 50 794 A1, DE 103 36 913 B4).

To produce dental restorations, it is known to press plastified ceramic material into a mold cavity present in a curable investment material (EP 1 484 031 B1, EP 0 231 773 A1).

DE 10 2015 108 171 A1 discloses a method to increase the strength of a form body consisting of lithium silicate glass ceramic. Lithium ions are replaced by alkali metal ions of greater diameter to generate a surface compressive stress.

The object of the present invention is to further develop a method of the type mentioned at the start in such a way that cracks and other surface defects that are seen during working, such as grinding, of the form body, are remedied. At the same time, a desired gloss effect is to be achieved if desired.

To solve this problem, the invention essentially provides the following:
- providing of a blank
- producing the form body by pressing and/or machining the blank
- softening of the form body exclusively in its surface region by irradiating the form body with infrared radiation over a time t, wherein main radiation components of the infrared radiation lie in the wavelength range between 2.7 µm and 4.7 µm, and the time t is 10 seconds ≤ t ≤ 150 seconds.

The form body should thereby be softened to a depth of T ≤ 100 µm, preferably T ≤ 50 µm, in particular T ≤ 10 µm.

Furthermore, the glass content of the lithium silicate glass ceramic should lie between 20% by volume and 60% by volume, in particular between 40 and 60% by volume. It is preferably provided that the ratio of the glass content to the crystal content is 45:55 percent by volume, in particular approximately 50:50 percent by volume.

The main crystal phase of the form body should be lithium disilicate. It is preferably provided that the size of the crystals or crystallites of the lithium silicate glass ceramic is below 2 µm. The composition should also be selected such that the TG (glass transition) temperature is < 800 °C.

In accordance with the invention, surface defects are remedied through the fact that the form body is softened exclusively on the surface, as a result of which, in particular, cracks are repaired. At the same time a gloss effect results due to the melting.

Since melting is confined to the surface, the form body retains its shape. In other words, the form body is heated in a short time in such a way that there is surface melting, i.e., temperatures occur which would lead to a deformation if a volume heating would take place.

Volume heating for ceramic materials is already known, for instance from DE 26 56 288 A1. In this process, a veneer ceramic is heated up to the full depth when it is applied to a metal or crown framework, to obtain a void-free veneering layer. At the same time, adhesion between metal and ceramic is to be improved. For volume heating, short-wave infrared radiation in the range between 0.7 and 1.5 µm is used.

To preclude unwanted volume heating, it is provided, in particular, for the surface heating to be carried out with an infrared radiation whose main radiation content is in particular between 35% and 50%.

The irradiation time should be in particular between 30 and 120 seconds.

The blank itself can already consist of lithium silicate glass ceramic. There is naturally also the possibility that, after the form body has been produced, it is still subjected to heat treatments in the customary manner to form metasilicate and/or disilicate crystals, in which case the disilicate crystals are present as the main crystal phase, especially after completion of the heat treatments.

If surface defects are to be healed in accordance with the prior art, a so-called healing fire conventional treatment can be carried out, i.e., an additional glaze is applied to the glass ceramic, which is then subjected to a heat treatment of approx. 12 minutes (time required for heating, holding and cooling).

On the other hand, due to the short exposure to radiation, the surface can be smoothed to a gloss very rapidly, without the radiation penetrating deeply. The dental restoration can be removed in the dental practice (chairside) for any grinding procedure that may be necessary for the correct fitting of the dental restoration to the residual teeth, the dental restoration is then reinserted after the short infrared irradiation according to the invention and cooling to body temperature.

This is preceded by etching of the inner surface of the dental restoration to insure adhesion to a preparation.

With regard to the blank, it should be noted that a blank is also to be understood as a pellet made of compacted powdery material which is used with known muffle systems to press a dental restoration.

Alternatively, the blank can be machined through the usual methods such as milling and grinding to provide the desired form body, in particular dental restoration. After processing, a healing on the surface and a smoothing to the gloss then take place within a very short time.

According to a further inventive proposal, the form body is covered, at least in sections, with a paste containing alkali metal ions of greater diameter than lithium ions before the infrared radiation. Thus, an ion exchange can take place during the infrared irradiation, so that not only is a smoothing / brightening or a gloss development brought about by the infrared radiation, but at the same time lithium ions are replaced by alkali metal ions of greater diameter so that a surface compressive stress and thus a strength increase is achievable.

In particular, provision is made for the form body to be coated with a viscous solution or dispersion of an alkali metal salt as the paste. Application through spraying or spreading is also possible.

The thickness of the paste should be at about 0.5 mm or less. It must be insured that infrared radiation can penetrate into the surface of the form body to a sufficient extent so that it can be absorbed and thus the form body can be heated on its surface to the required extent.

In particular, the invention is distinguished by the fact that the lithium silicate glass ceramic contains, in percentage by weight, in its starting composition:

| | |
|---|---|
| SiO₂ | 54.0 - 62.0, preferably 57.0 - 62.0 |
| Nucleating agent, such as P₂O₅ | 5.0 - 6.0 |
| Al₂O₃ | 1.5 - 3.5 |
| Li₂O | 13.0 - 16.0 |
| K₂O | 0.6 - 1.8 |
| ZrO₂ | 8.0 - 11.5 |
| B₂O₃ | 0 - 6.0 |
| Na₂O | 0 - 1.9 |
| Color pigments | 0 - 8.0 |
| such as MnO, Fe₂O₃, Tb₂O₃, Er₂O₃, Pr₂O₃, CeO₂, Y₂O₃, V₂O₃ | |

The lithium silicate glass ceramic of the following starting composition in percentage by weight is preferred:

| | |
|---|---|
| SiO₂ | 57.0 - 60.0 |
| Nucleating agent, such as P₂O₅ | 5.2 - 5.6 |
| Al₂O₃ | 2.6 - 3.2 |
| Li₂O | 13.5 - 15.0 |
| K₂O | 0.8 - 1.4 |
| ZrO₂ | 9.0 - 11.0 |
| B₂O₃ | 0 - 5.0 |
| Na₂O | 0 - 1.5 |
| Color pigments | 2 - 7.0 |
| (CeO₂ may also be used as a color pigment) | |

The lithium silicate glass ceramic of the following starting components in percentage by weight is especially preferred:

| | |
|---|---|
| SiO₂ | 58 |
| P₂O₅ | 5 |
| Al₂O₃ | 3 |
| Li₂O | 15 |
| K₂O | 1 |
| ZrO₂ | 10.0 |
| Color pigment(s) | 4 |
| such as MnO, Fe₂O₃, Tb₂O₃, Er₂O₃, Pr₂O₃, Y₂O₃, V₂O₃ | |
| Additives | 4 |
| such as Na₂O, nucleating agent, such as B₂O₃ or refining agent such as CeO₂ | |

In an embodiment, the invention is characterized in that the blank or the form body is subjected at least to a first heat treatment W1 at a temperature T_{W1} over a period t_{W1}, wherein 620 °C ≤ T_{W1} ≤ 800 ° C, in particular 650 °C ≤ T_{W1} ≤ 750 ° C, and/or 1 minute ≤ t_{W1} ≤ 200 minutes, preferably 10 minute ≤ tW1 ≤ 60 minutes. If the blank is heat-treated, the form body is then produced therefrom.

A corresponding lithium silicate glass ceramic blank can be worked without difficulty, with minimal tool wear. A corresponding blank can also be pressed into a desired geometry.

In particular, in order to achieve a final crystallization, the lithium silicate glass ceramic blank, or the form body, is subjected to a second heat treatment W2 at a temperature T_{W2} for a time t_{W2} after the first heat treatment W1, wherein 800 °C ≤ T_{W2} ≤ 1040 °C, 800 °C ≤ T_{W2} ≤ 900 °C and/or 2 minutes ≤ t_{W2} ≤ 200 minutes, preferably 3 minutes ≤ t_{W2} ≤ 30 minutes.

The following temperature values and heating rates are preferably selected in the heat treatment steps leading to a pre-crystallization or final crystallization. With respect to the first heat treatment W1, it is provided in particular that this takes place in two stages, with a first holding stage in the range between 640 °C and 680 °C and a second holding stage between 720 °C and 780 °C. In each stage, the heated blank is held for a period of time, preferably between 35 and 45 minutes in the first stage and preferably between 15 and 25 minutes in the second stage.

The form body is preferably covered, at least over regions, with a paste containing potassium ions, in particular with a paste containing KNO₃, KCl or K₂CO₃, or with a paste containing sodium ions, in particular with a paste containing NaNO₃, sodium acetate or sodium salts of organic acids, or with a paste containing a mixture of potassium ions and sodium ions, in particular in the ratio 50:50 mol.%, preferably with a paste containing NaNO₃ and KNO₃.

Further details, advantages and features of the invention result not only from the claims, the characteristics to be drawn from them-both on their own and/or in combination-but also from the following description of preferred embodiments The scope of the invention is defined by the claims.

For the production of blanks, the required raw materials were first melted over a period of more than two hours at 1540 °C. According to the manufacturer, the following starting composition was used in percentage by weight to prepare lithium silicate glass and from this lithium silicate glass ceramic.

| | |
|---|---|
| SiO₂ | 58.1 - 59.1 |
| P₂O₅ | 5.8 - 5.9 |
| Al₂O₃ | 1.9 - 2.0 |
| Li₂O | 18.5 - 18.8 |
| K₂O | 1.9 - 2.0 |
| ZrO₂ | 9.5 - 10.5 |
| CeO₂ | 1.0 - 2.0 |
| Tb₄O₇ | 1.0 - 1.5 |
| Na₂O | 0 - 0.2 |

The melt was then filled into containers, the filling temperature of the melt being 1360 °C. The temperature within the containers was approximately in the range 800 °C to 1250 °C. The melt was then cooled to 300 °C to 500 °C in the containers. Subsequently, a slow relaxation to the room temperature was carried out over a period of 2 hours.

Then, a three-stage heat treatment was performed to form crystal phases. In a first crystallization step, the blanks were held at a temperature of 530 °C for 30 minutes. In a second step, they were heated to 670 °C and held at this temperature for almost 120 minutes. In a third step, the blanks were held at 800 °C for 30 minutes. This was followed by cooling to room temperature. Analysis showed that lithium disilicate is contained as the main crystal phase in the blanks. The crystal phase is in a ratio of approximately 1:1 to the glass content in percentage by volume. The size of the crystals / crystallites in the glass ceramic was a maximum of 2 µm.

A crown was then prepared chairside. For this purpose, a tooth preparation of a patient was scanned and the dental restoration was then milled from the blank using a CAD / CAM method. The dental restoration was then fitted and adapted. The crown was then removed to bring about smoothing to the gloss and healing of the damaged surface according to the teaching of the invention.

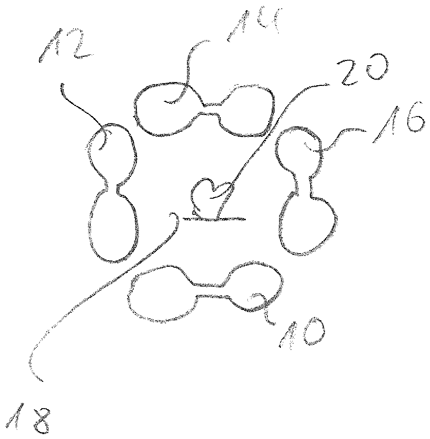

For this purpose, the dental restoration was exposed to infrared radiation emitted by quartz radiators, with the radiation component in the wavelength range between 2.5 and 4.7 µm being approximately 40%. Elongated quartz radiators 10, 12, 14, 16 were used, which in section formed an eight, as can be seen from the single figure. Corresponding radiators 10, 12, 14, 16 delimited a parallelepiped space 18, in which the crown 20 was positioned. The crown 20 was exposed to infrared radiation over a period of about 60 seconds. In this case, heating alone was carried out, which led exclusively to a melting of the surface. This heating of the surface alone resulted in remediation of the damaged surface and in particular the cracks therein. At the same time, a gloss effect was achieved.

Since only a surface melting took place, cooling was rapid, so that the crown 20 could be reused after just approximately 1 min. Previously, the inner surface of the crown 20 was roughened by etching. Finally the crown was positioned and cemented in place.

Thus, a chairside treatment could be carried out in a very short period of time.

According to the teachings of the invention, a lithium silicate glass ceramic with a glass content of between 30 and 65% by volume and crystallite sizes in the glass ceramic of up to 2 µm and irradiation with an infrared radiation of between 2.5 µm and 4 µm is softened on the surface so that surface cracks or other damage that result upon surface treatment, such as milling, are repaired. At the same time, a gloss surface is obtained.

The strength is increased in a very short time, as possible surface damage is cured.

The glaze application according to the prior art, which is time-consuming, and the subsequent firing process are not required.

Optionally, it is possible to increase the strength by replacing lithium ions with alkali metal ions of greater diameter during treatment with infrared radiation. For this purpose, in the areas in which a strength increase is to be achieved, the form body, such as a dental restoration, is to be coated with a corresponding paste containing the alkali metal ions.

Although the teaching according to the invention preferably applies to the field of dental technology, it is not restricted to that field, but rather applies to all applications in which molded parts are produced from lithium silicate glass ceramic, in particular also in other medical fields.

## Claims

1. A method for the production of a monolithic form body (20), in particular a dental restoration such as a crown, bridge, coping, inlay, onlay or veneer, of lithium silicate glass ceramic comprising the steps of:
- providing a blank
- producing the form body by pressing and/or machining the blank
- softening of the form body exclusively in the surface region by irradiating the form body with infrared radiation over a time t, wherein main radiation components of the infrared radiation lie in the wavelength range between 2.7 µm and 4.7 µm, and the time t is 10 seconds ≤ t ≤ 150 seconds.

2. The method according to claim 1, wherein the form body (20) is softened to a depth T of T ≤ 100 µm, preferably T ≤ 50 µm, in particular T ≤ 10 µm.

3. The method according to claim 1 or 2, wherein the form body (20) is irradiated with the infrared radiation with main radiation content between 35% and 50%.

4. The method according to at least one of the preceding claims, wherein a blank of lithium silicate glass ceramic is used which contains lithium disilicate as the main crystal phase.

5. The method according to at least one of the preceding claims, wherein the form body (20) is heat-treated after its production for the formation of metasilicate and/or disilicate crystals, in particular disilicate crystals, as the main crystal phase.

6. The method according to at least one of the preceding claims, wherein a form body (20) of lithium silicate glass ceramic is used, wherein the glass content of said lithium silicate glass ceramic lie between 20% by volume and 60% by volume, in particular between 40 and 60% by volume; and/or wherein the ratio of the glass content to the crystal content is 45:55 percent by volume, in particular 50:50 percent by volume.

7. The method according to at least one of the preceding claims, wherein the size of the crystals or crystallites of the lithium silicate glass ceramic is below 2 µm.

8. The method according to at least one of the preceding claims, wherein the form body (20) is irradiated with the infrared radiation over a time t where 30 seconds ≤ t ≤ 120 seconds.

9. The method according to at least one of the preceding claims, wherein the lithium silicate glass ceramic in its starting composition, contains or consists of the following in percentage by weight:
| | |
|---|---|
| SiO₂ | 54.0 - 62.0, preferably 57.0 - 62.0 |
| Nucleating agents, such as P₂O₅ | 5.0 - 6.0 |
| Al₂O₃ | 1.5 - 3.5 |
| Li₂O | 13.0 - 16.0 |
| K₂O | 0.6 - 1.8 |
| ZrO₂ | 8.0 - 11.5 |
| B₂O₃ | 0 - 6.0 |
| Na₂O | 0 - 1.9 |
| Color pigments | 0 - 8.0 |
| such as MnO, Fe₂O₃, Tb₂O₃, Er₂O₃, Pr₂O₃, CeO₂, Y₂O₃, V₂O₃ | |

10. The method according to at least one of the preceding claims, wherein the lithium silicate glass ceramic in its starting composition, contains or consists of the following in percentage by weight:
| | |
|---|---|
| SiO₂ | 57.0 - 60.0 |
| Nucleating agents such as P₂O₅ | 5.2 - 5.6 |
| Al₂O₃ | 2.6 - 3.2 |
| Li₂O | 13.5 - 15.0 |
| K₂O | 0.8 - 1.4 |
| ZrO₂ | 9.0 - 11.0 |
| B₂O₃ | 0 - 5.0 |
| Na₂O | 0 - 1.5 |
| Color pigments | 2 - 7.0 |
wherein Ce02 may also be contained.

11. The method according to at least one of the preceding claims, wherein the lithium silicate glass ceramic is derived from the following starting components in percentage by weight:
| | |
|---|---|
| SiO₂ | 58 |
| P₂O₅ | 5 |
| Al₂O₃ | 3 |
| Li₂O | 15 |
| K₂O | 1 |
| ZrO₂ | 10.0 |
| Color pigment(s) | 4 |
| such as MnO, Fe₂O₃, Tb₂O₃, Er₂O₃, Pr₂O₃, Y₂O₃, V₂O₃ | |
| Additives (refining agent and nucleating agent) | 4 |
| such as B₂O₃, CeO₂, Na₂O | |

12. A method preferably according to one of the preceding claims, wherein the form body (20) is covered, at least in sections, with a paste containing alkali metal ions of greater diameter than lithium ions before subjected to its infrared irradiation.

13. The method according to claim 12, wherein the form body (20) is coated with a viscous solution or dispersion of a salt containing an alkali metal ion as the paste; wherein preferably to derive the paste the salt is mixed with at least one substance from the group consisting of 1,4-butanediol, hexanetriol, or a mixture of the two substances.

14. The method according to claim 12, wherein the paste is applied to the form body (20) by spraying and/or the paste is applied to all the surfaces of the form body, in particular with a thickness D of about 0.5 mm or less, in particular of 0.1 mm < D < 0.4 mm.

## Patentansprüche

1. Verfahren zur Herstellung eines monolithischen Formkörpers (20), insbesondere einer Zahnrestauration wie einer Krone, einer Brücke, einer Kappe, eines Inlays, eines Onlays oder einer Verblendung aus Lithiumsilicatglaskeramik, umfassend die Schritte:
- Bereitstellen eines Rohlings
- Herstellen des Formkörpers durch Pressen und/oder Bearbeiten des Rohlings
- Erweichen des Formkörpers ausschließlich im Oberflächenbereich durch Bestrahlen des Formkörpers mit Infrarotstrahlung über eine Zeit t, wobei die Hauptstrahlungskomponenten der Infrarotstrahlung im Wellenlängenbereich zwischen 2,7 µm und 4,7 µm liegen und die Zeit t 10 Sekunden ≤ t ≤ 150 Sekunden beträgt.

2. Verfahren nach Anspruch 1, wobei der Formkörper (20) auf eine Tiefe T von T ≤ 100 µm, vorzugsweise T ≤ 50 µm, insbesondere T ≤ 10 µm, erweicht wird.

3. Verfahren nach Anspruch 1 oder 2, wobei der Formkörper (20) mit der Infrarotstrahlung mit einem Hauptstrahlungsgehalt zwischen 35 % und 50 % bestrahlt wird.

4. Verfahren nach mindestens einem der vorhergehenden Ansprüche, wobei ein Rohling aus Lithiumsilicatglaskeramik verwendet wird, der Lithiumdisilicat als Hauptkristallphase enthält.

5. Verfahren nach mindestens einem der vorhergehenden Ansprüche, wobei der Formkörper (20) nach dessen Herstellung zur Bildung von Metasilicat- und/oder Disilicatkristallen, insbesondere Disilicatkristallen, als Hauptkristallphase wärmebehandelt wird.

6. Verfahren nach mindestens einem der vorhergehenden Ansprüche, wobei ein Formkörper (20) aus Lithiumsilicatglaskeramik verwendet wird, wobei der Glasgehalt der Lithiumsilicatglaskeramik zwischen 20 Vol.-% und 60 Vol.-%, insbesondere zwischen 40 und 60 Vol.-% liegt; und/oder wobei das Verhältnis des Glasgehalts zum Kristallgehalt 45:55 Vol.-%, insbesondere 50:50 Vol.-% beträgt.

7. Verfahren nach mindestens einem der vorhergehenden Ansprüche, wobei die Größe der Kristalle oder Kristallite der Lithiumsilicatglaskeramik unter 2 µm liegt.

8. Verfahren nach mindestens einem der vorhergehenden Ansprüche, wobei der Formkörper (20) mit der Infrarotstrahlung über eine Zeit t bestrahlt wird, wobei 30 Sekunden ≤ t ≤ 120 Sekunden.

9. Verfahren nach mindestens einem der vorhergehenden Ansprüche, wobei die Lithiumsilicatglaskeramik in deren Ausgangszusammensetzung Folgendes in Gewichtsprozent enthält oder daraus besteht:
| | |
|---|---|
| SiO₂ | 54,0-62,0, vorzugsweise 57,0-62,0 |
| Keimbildner wie P₂O₅ | 5,0-6,0 |
| Al₂O₃ | 1,5-3,5 |
| Li₂O | 13,0-16,0 |
| K₂O | 0,6-1,8 |
| ZrO₂ | 8,0-11,5 |
| B₂O₃ | 0-6,0 |
| Na₂O | 0-1,9 |
| Farbpigmente | 0-8,0 |
| z. B. MnO, Fe₂O₃, Tb₂O₃, Er₂O₃, Pr₂O₃, CeO₂, Y₂O₃, V₂O₃ | |

10. Verfahren nach mindestens einem der vorhergehenden Ansprüche, wobei die Lithiumsilicatglaskeramik in deren Ausgangszusammensetzung Folgendes in Gewichtsprozent enthält oder daraus besteht:
| | |
|---|---|
| SiO₂ | 57,0-60,0 |
| Keimbildner wie z. B. P₂O₅ | 5,2-5,6 |
| Al₂O₃ | 2,6-3,2 |
| Li₂O | 13,5-15,0 |
| K₂O | 0,8-1,4 |
| ZrO₂ | 9,0-11,0 |
| B₂O₃ | 0-5,0 |
| Na₂O | 0-1,5 |
| Farbpigmente | 2-7,0 |
wobei auch CeO₂ enthalten sein kann.

11. Verfahren nach mindestens einem der vorhergehenden Ansprüche, wobei die Lithiumsilicatglaskeramik von den folgenden Ausgangskomponenten in Gewichtsprozent abgeleitet ist:
| | |
|---|---|
| SiO₂ | 58 |
| P₂O₅ | 5 |
| Al₂O₃ | 3 |
| Li₂O | 15 |
| K₂O | 1 |
| ZrO₂ | 10,0 |
| Farbpigment(e) | 4 |
| wie z. B. MnO, Fe₂O₃, Tb₂O₃, Er₂O₃, Pr₂O₃, Y₂O₃, V₂O₃ | |
| Zusatzstoffe (Raffiniermittel und Keimbildner) | 4 |
| wie z. B. B₂O₃, CeO₂, Na₂O | |

12. Verfahren vorzugsweise nach einem der vorhergehenden Ansprüche, wobei der Formkörper (20) mindestens abschnittsweise mit einer Paste bedeckt ist, die Alkalimetallionen mit größerem Durchmesser als Lithiumionen enthält, bevor sie dessen Infrarotbestrahlung unterzogen wird.

13. Verfahren nach Anspruch 12, wobei der Formkörper (20) mit einer viskosen Lösung oder Dispersion eines Salzes beschichtet ist, das ein Alkalimetallion als Paste enthält; wobei vorzugsweise zur Ableitung der Paste das Salz mit mindestens einer Substanz aus der Gruppe gemischt ist, die aus 1,4-Butandiol, Hexantriol oder einer Mischung der zwei Substanzen besteht.

14. Verfahren nach Anspruch 12, wobei die Paste durch Sprühen auf den Formkörper (20) aufgebracht wird und/oder die Paste auf alle Oberflächen des Formkörpers aufgebracht wird, insbesondere mit einer Dicke D von etwa 0,5 mm oder weniger, insbesondere von 0,1 mm < D < 0,4 mm.

## Revendications

1. Procédé de production d'un corps à forme monolithique (20), en particulier une restauration dentaire telle qu'une couronne, un bridge, une coiffe, un inlay, un onlay ou une facette, en vitrocéramique au silicate de lithium, comprenant les étapes consistant à :
- utiliser une ébauche
- produire le corps à forme par pressage et/ou par usinage de l'ébauche
- ramollir le corps à forme exclusivement dans la région de surface par exposition du corps à forme au rayonnement infrarouge pendant un temps t, les principaux composants du rayonnement infrarouge se situant dans la plage de longueurs d'onde comprise entre 2,7 µm et 4,7 µm, et le temps t étant de 10 secondes ≤ t ≤ 150 secondes.

2. Procédé selon la revendication 1, le corps à forme (20) étant ramolli à une profondeur T, T ≤ 100 µm, de préférence T ≤ 50 µm, en particulier T ≤ 10 µm.

3. Procédé selon la revendication 1 ou 2, le corps à forme (20) étant exposé au rayonnement infrarouge dont le taux de rayonnement principal est compris entre 35 % et 50 %.

4. Procédé selon au moins l'une des revendications précédentes, une ébauche en vitrocéramique de silicate de lithium contenant du disilicate de lithium comme phase cristalline principale, est utilisée.

5. Procédé selon au moins l'une des revendications précédentes, le corps à forme (20) étant traité thermiquement après sa production pour la formation de cristaux de métasilicate et/ou de disilicate, en particulier de cristaux de disilicate, comme phase cristalline principale.

6. Procédé selon au moins l'une des revendications précédentes, la teneur en verre de ladite vitrocéramique de silicate de lithium du corps à forme (20) en vitrocéramique de silicate de lithium utilisé étant compris entre 20 % en volume et 60 % en volume, en particulier entre 40 et 60 % en volume ; et/ou le rapport entre la teneur en verre et la teneur en cristaux étant de 45:55 pour cent en volume, en particulier 50:50 pour cent en volume.

7. Procédé selon au moins l'une des revendications précédentes, la taille des cristaux ou des cristallites de la vitrocéramique de silicate de lithium étant inférieure à 2 µm.

8. Procédé selon au moins l'une des revendications précédentes, le corps à forme (20) étant exposé au rayonnement infrarouge pendant un temps t, 30 secondes ≤ t ≤ 120 secondes.

9. Procédé selon au moins l'une des revendications précédentes, la vitrocéramique de silicate de lithium dans sa composition de départ contenant ou étant constituée des éléments suivants en pourcentage en poids :
| | |
|---|---|
| SiO₂ | 54,0 - 62,0, de préférence 57,0 - 62,0 |
| Agents de nucléation, tels que P₂O₅ | 5,0-6,0 |
| Al₂O₃ | 1,5-3,5 |
| Li₂O | 13,0-16,0 |
| K₂O | 0,6-1,8 |
| ZrO₂ | 8,0-11,5 |
| B₂O₃ | 0-6,0 |
| Na₂O | 0-1,9 |
| Pigments de couleur | 0-8,0 |
| tels que MnO, Fe₂O₃, Tb₂O₃, Er₂O₃, Pr₂O₃, CeO₂, Y₂O₃, V₂O₃ | |

10. Procédé selon au moins l'une des revendications précédentes, la vitrocéramique de silicate de lithium dans sa composition de départ contenant ou étant constituée des éléments suivants en pourcentage en poids :
| | |
|---|---|
| SiO₂ | 57,0-60,0 |
| Agents de nucléation tels que P₂O₅ | 5,2-5,6 |
| Al₂O₃ | 2,6-3,2 |
| Li₂O | 13,5-15,0 |
| K₂O | 0,8-1,4 |
| ZrO₂ | 9,0-11,0 |
| B₂O₃ | 0-5,0 |
| Na₂O | 0-1,5 |
| Pigments de couleur | 2-7,0 |
dans laquelle CeO₂ peut également être contenu.

11. Procédé selon au moins l'une des revendications précédentes, la vitrocéramique de silicate de lithium étant dérivée des composants de départ suivants en pourcentage en poids :
| | |
|---|---|
| SiO₂ | 58 |
| P₂O₅ | 5 |
| Al₂O₃ | 3 |
| Li₂O | 15 |
| K₂O | 1 |
| ZrO₂ | 10,0 |
| Pigment(s) de couleur | 4 |
| tels que MnO, Fe₂O₃, Tb₂O₃, Er₂O₃, Pr₂O₃, Y₂O₃, V₂O₃ | |
| Additifs (agent de raffinage et agent de nucléation) | 4 |
| tels que B₂O₃, CeO₂, Na₂O | |

12. Procédé de préférence selon l'une des revendications précédentes, le corps à forme (20) étant recouvert, au moins à certains endroits, d'une pâte contenant des ions de métal alcalin de diamètre supérieur à celui des ions lithium avant son exposition au rayonnement infrarouge.

13. Procédé selon la revendication 12, le corps à forme (20) étant revêtu d'une solution visqueuse ou d'une dispersion d'un sel contenant un ion de métal alcalin comme pâte ; de préférence, pour obtenir la pâte, le sel étant mélangé avec au moins une substance du groupe constitué de butane-1,4-diol, hexanetriol, ou un mélange des deux substances.

14. Procédé selon la revendication 12, la pâte étant appliquée sur le corps à forme (20) par pulvérisation et/ou la pâte étant appliquée sur toutes les surfaces du corps à forme, en particulier d'une épaisseur D inférieure ou égale à environ 0,5 mm, en particulier 0,1 mm < D < 0,4 mm.
